(19) Europäisches Patentamt
European Patent Office
Office européen des brevets

(11) Publication number: **0 006 352 A1**

# (12) EUROPEAN PATENT APPLICATION

(21) Application number: 79301143.8

(22) Date of filing: 14.06.79

(51) Int. Cl.³: C 07 D 233/76, A 61 K 31/415

(30) Priority: 15.06.78 GB 2701278

(43) Date of publication of application: 09.01.80 Bulletin 80/1

(84) Designated Contracting States: BE CH DE FR GB IT NL SE

(71) Applicant: BEECHAM GROUP LIMITED, Beecham House Great West Road, Brentford, Middlesex (GB)

(72) Inventor: Wootton, Gordon, 51 Park Way, Sawbridgeworth, Hertfordshire (GB)
Inventor: Moore, Richard William, 34 Sunningdale, Bishops Stortford Hertfordshire (GB)

(74) Representative: Dawson, Hugh Bainforde, Dr. et al, BEECHAM PHARMACEUTICALS Yew Tree Bottom Road, Epsom, Surrey KT18 5XQ. (GB)

## (54) Hydantoins and thiohydantoins, process for their preparation and pharmaceutical compositions containing them.

(57) A compound of the formula (I):

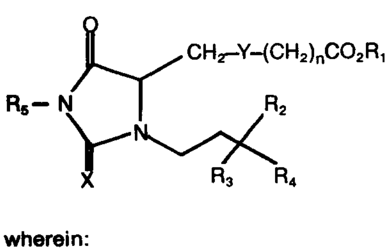

(I)

wherein:

X is O or S;

Y is $-CH=CH-$ or $-C \equiv C-$;

n is 1 to 5;

$R_1$ is hydrogen or $CO_2R_1$ represents an ester group in which the $R_1$ moiety contains from 1 to 12 carbon atoms;

$R_2$ is hydrogen or $C_{1-4}$ alkyl;

$R_3$ is hydroxy or protected hydroxy;

$R_4$ is $C_{1-9}$ alkyl, $C_{3-8}$ cycloalkyl or $C_{3-8}$ cycloalky-$C_{1-6}$ alkyl; or

$R_2$ and $R_4$ taken with the carbon atom to which they are joined represent a $C_{5-8}$ cycloalkyl group; and

$R_5$ is $C_{1-6}$ alkyl substituted by one nitro, hydroxy, $C_{1-6}$ alkoxy, $CO_2A$, $(CO_2A)_2$, CN or halogen group; $C_{5-8}$ cycloalkyl; or $CO_2A$; in $R_5$ when present A is hydrogen or $CO_2A$ represents an ester group in which the A moiety contains from 1 to 12 carbon atoms; and salts thereof; has useful pharmaceutical activity, including bronchodilator activity.

EP 0 006 352 A1

TITLE MODIFIED
see front page

# Prostaglandin Analogues

This invention relates to novel compounds having pharamacological activity, to a process for their preparation, to intermediates useful in that process and to pharmaceutical compositions containing them.

German Offenlegungsschrift No. 2724948 dicloses that compounds of the general formula (A):

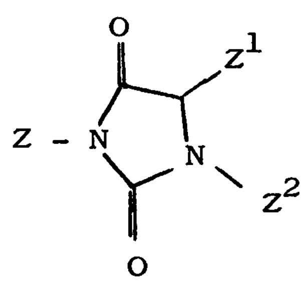

(A)

wherein Z is hydrogen or alkyl; one of $Z^1$ and $Z^2$ is a group - $CH_2$-X-$X^1$-$X^2$ in which X is phenylene, -C≡C-, <u>cis</u>- or <u>trans</u>- -CH=CH- or -$CH_2$-$CQ_2$-, where each radical Q independently of the other is hydrogen or alkyl or the two radicals Q together are $C_{4-6}$ alkylene, $X^1$ is a covalent bond or a straight or branched $C_{1-6}$ alkylene chain, in which one methylene group is optionally substituted by an oxa (-O-) group, with the proviso that at least one carbon atom separates the oxa group from a -C≡C-, -CH=CH- or CO group, and $X^2$ is tetrazolyl,

carboxyl, carboxamide, hydroxymethylene or alkoxycarbonyl; and the other one of $Z^1$ and $Z^2$ is a group $-Y-Y^1-Y^2-Y^3$ in which Y is $-CR_2-CH_2-$, where each radical R independently of the other is hydrogen or methyl, $Y^1$ is carbonyl, methylene, methylene substituted by a hydroxy group or methylene substituted by a hydroxy and alkyl group, $Y^2$ is a covalent bond or straight-chain or branched $C_{1-7}$ alkylene optionally substituted on the carbon atom adjacent to $Y^1$ by one or two mutually independent alkyl, bicycloalkyl or cycloalkyl groups, $Y^3$ is hydrogen, hydroxy, $C_{1-7}$(preferably $C_{1-4}$)alkoxy, cycloalkyl, bicycloalkyl, phenyl, benzyl, phenoxy or benzyloxy, where each phenyl, benxyl, phenoxy or benzyloxy group may be substituted in the benzene ring by one or more hydroxy, halogen, nitro, amino, acylamino, alkenyl, alkoxy, phenyl or alkyl groups, which themselves may be substituted by one or more halogens or Y is a bond, $-CH_2-$ or $-CH_2.CH_2-$ and $Y^1$, $Y^2$ and $Y^3$ together are cycloalkyl which is substituted by a hydroxy group which is preferably separated by 3 carbon atoms from the hydantoin ring, have similar pharmacological activity to natural prostaglandins.

We have now discovered a class of compounds which have useful pharmocological activity and which are structurally distinct from the compounds disclosed in Offenlegungsschrift No. 2724948.

This class of compounds of this invention is structurally distinct from the compounds of formula (B):

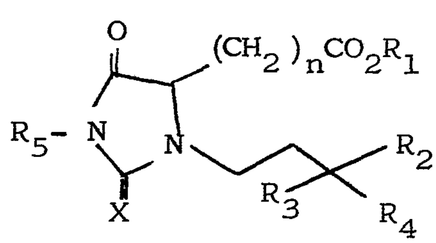

(B)

0006352



wherein:

X is O to S;

n is 1 to 8;

$R_1$ is hydrogen, or $CO_2R_1$ represents an ester group in which the $R_1$ moiety contains from 1 to 12 carbon atoms;

$R_2$ is hydrogen, $C_{1-4}$ alkyl, or phenyl;

$R_3$ is hydroxy or protected hydroxy;

$R_4$ is hydrogen, $C_{1-9}$ alkyl, $C_{3-8}$ cycloalkyl, $C_{3-8}$ cycloalkyl-$C_{1-6}$ alkyl, phenyl, phenyl $C_{1-6}$ alkyl, naphthyl, naphthyl-$C_{1-6}$-alkyl, any of which phenyl or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups; or

$R_2$ and $R_4$ taken with the carbon atom to which they are joined represent a $C_{5-8}$ cycloalkyl groups;

$R_5$ is hydrogen, $C_{1-6}$ alkyl, $C_{1-6}$ alkyl substituted by a nitro, hydroxy, $C_{1-6}$ alkoxy, $CO_2A$, $(CO_2A)_2$, CN or halogen group, $C_{5-8}$ cycloalkyl, phenyl, phenyl-$C_{1-6}$ alkyl, phenyl-$C_{3-6}$ cycloalkyl, any of which phenyl moieties may be substituted by one or more halogen, trifluoromethyl, $C_{1-6}$ alkyl, $C_{1-6}$alkoxy or nitro groups; or a group $CO_2A$; in R when present A is hydrogen or $CO_2A$ represents an ester group in which the A moiety contains from 1 to 12 carbon atoms; and salts thereof; which are disclosed in our West German Offenlegungsschrift No. 2755771 as having useful prostaglandin-like activity. It should be noted that West German Offenlegungsscrift No. 2755771 published after the date of filing of the priority application for this invention.

Accordingly the present invention provides a compound of formula (I):

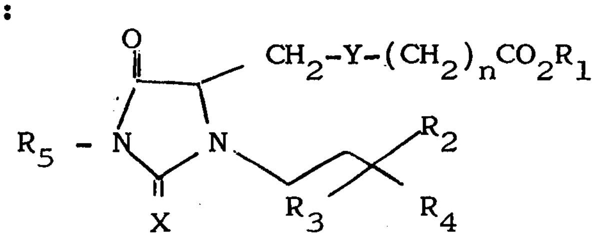

(I)

wherein:

X is O or S;

Y is -CH=CH- or -C≡C-;

n is 1 to 5;

$R_1$ is hydrogen or $CO_2R_1$ represents an ester group in which the $R_1$ moiety contains from 1 to 12 carbon atoms;

$R_2$ is hydrogen or $C_{1-4}$ alkyl;

$R_3$ is hydroxy or protected hydroxy;

$R_4$ is $C_{1-9}$ alkyl, $C_{3-8}$ cycloalkyl or $C_{3-8}$ cycloalkyl - $C_{1-6}$ alkyl; or

$R_2$ and $R_4$ taken with the carbon atom to which they are joined represent a $C_{5-8}$ cycloalkyl group; and

$R_5$ is $C_{1-6}$ alkyl substituted by one nitro, hydroxy, $C_{1-6}$ alkoxy, $CO_2A$, $(CO_2A)_2$, CN or halogen group; $C_{5-8}$ cycloalkyl; or $CO_2A$; in $R_5$ when present A is hydrogen or $CO_2A$ represents an ester group in which the A moiety contains from 1 to 12 carbon atoms; and salts thereof.

Suitable examples of $R_1$ include hydrogen, methyl, ethyl, n- and iso-propyl, n-, sec- and tert-butyl, phenyl, benzyl, tolyl and the like, while normally hydrogen or $C_{1-6}$ alkyl groups are preferred.

Suitable examples of $R_2$ include hydrogen, methyl and ethyl. More suitably $R_2$ is hydrogen or methyl, preferably methyl.

Suitable protected hydroxyl groups $R_3$ include readily hydrolysable groups such as acylated hydroxy groups in which the acyl moiety contains 1 to 4 carbon atoms, for example the acetoxy group; and hydroxy groups etherified by readily removable inert groups such as the benzyl group or like groups. Preferably $R_3$ is hydroxyl.

Suitable groups $R_4$ when $R_4$ is an alkyl group include $C_{4-9}$ alkyl groups. Such $C_{4-9}$ alkyl groups may be straight chain alkyl groups, such as n-butyl, n-pentyl, n-hexyl and n-heptyl, or may be alkyl groups branched by one or two methyl groups (at the same or different carbon atoms). Thus for example, $R_4$ may be a group $CH_2R_7$, $CH(CH_3)R_7$ or $C(CH_3)_2R_7$, wherein $R_7$ is a straight chain alkyl group such that the carbon content of the resultant group $R_4$ is 4 to 9.

In general preferred groups $R_4$ when $R_4$ is an alkyl group include straight chain pentyl, hexyl and heptyl groups. Of these straight chain hexyl is often the most useful. Other preferred groups $R_4$ include groups $CH(CH_3)R_7$ and $C(CH_3)_2R_7$ wherein $R_7$ is straight chain butyl, pentyl and hexyl.

Other suitable examples of $R_4$ when $R_4$ is an alkyl group include the lower alkyl groups, that is when $R_4$ is a $C_{1-4}$ alkyl group.

When $R_4$ is or contains a $C_{3-8}$ cycloalkyl moiety, the moiety may be cyclopropyl. The moiety may also be a $C_{5-8}$ cycloalkyl moiety such as a cyclohexyl moiety. Examples of suitable $C_{1-6}$ alkyl moieties when $R_4$ is a $C_{3-8}$ cycloalkyl $C_{1-6}$ alkyl group include methyl, ethyl, propyl, butyl and pentyl.

Also $R_2$ and $R_4$ taken with the carbon atom to which they are joined can represent a $C_{5-8}$ cycloalkyl group, such as the cyclohexyl group.

When $R_5$ is a $C_{1-6}$ alkyl group substituted by a nitro, hydroxy, $C_{1-6}$ alkoxy (such as methoxy), $CO_2A$, $(CO_2A)_2$, CN or halogen, often $R_5$ will be a methylene group thus substituted.

When $R_5$ is, or contains, a group $CO_2A$, suitable examples of A include hydrogen, methyl, ethyl, n- and iso-propyl, n-, sec- and tert-butyl, phenyl, benzyl, toluyl and the like, while normally for A hydrogen or $C_{1-6}$ alkyl are preferred.

When $R_5$ is $C_{5-8}$ cycloalkyl, it is suitably cyclohexyl.

Preferred $R_5$ groups include $C_{1-6}$ alkyl substituted by CN, such as $-CH_2CN$. Suitably n is 2 to 4, preferably 3.

The compounds of the formula (I) may form conventional salts. Such salts include those with alkali and alkaline earth metals, suitably sodium and potassium, and ammonium and substituted ammonium salts.

Form the aforesaid it will be seen that one particularly suitable group of compounds within formula (I) is of formula (II):

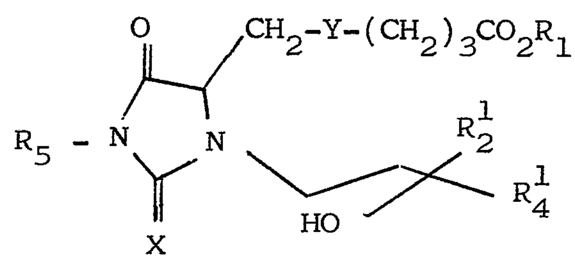

(II)

wherein:

X, Y, $R_1$ and $R_5$ are as defined in formula (I);

$R_2^1$ is hydrogen, methyl or ethyl;

$R_4^1$ is $C_{1-9}$ alkyl; and salts thereof.

In formula (II) suitably X is O.

$R_2^1$ is more suitably hydrogen or methyl, preferably methyl.

While $R_4^1$ may be a $C_{1-9}$ alkyl group, it is normally a $C_{4-9}$ alkyl group. In such cases suitable and preferred straight chain and branched groups $R_4^1$ include those previously described as suitable and preferred for the group $R_4$ when $R_4$ is a $C_{4-9}$ alkyl group. Such preferred groups $R_4^1$ include straight chain pentyl, hexyl, and heptyl and of these normally the most useful is straight chain hexyl. Other preferred groups $R_4^1$ include $CH(CH_3)R_7^1$ and $C(CH_3)_2R_7^1$ wherein $R_7^1$ is straight chain butyl, pentyl or hexyl.

Suitable and preferred values for X, Y, $R_1$ and $R_5$ are as described hereinbefore.

A further group of compounds within formula (I)

of interest is of formula (III):

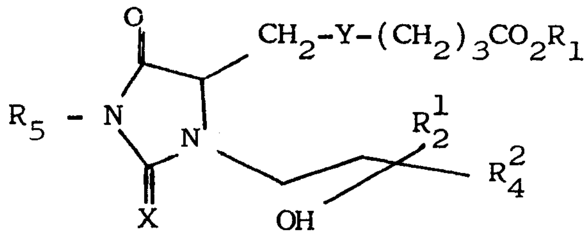

(III)

wherein:

X, Y, $R_1$ and $R_5$ are as defined in formula (I);

$R_2^1$ is hydrogen, methyl or ethyl;

$R_4^2$ is a group of formula (IV);

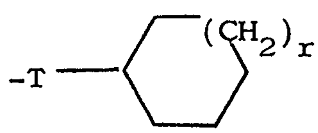

(IV)

wherein T is a bond, or a $C_{1-6}$ alkylene group which may be straight chain or branched by one or two methyl groups at the same or different carbon atoms; and

r is 0 to 3;

and salts thereof.

In formula (III) suitably X is O.

$R_2^1$ is more suitably hydrogen or methyl, preferably methyl.

In formula (IV) often T will be a group $-(CH_2)_q-$ wherein q is 0 to 4. Also suitably r is 1.

It will of course be realised that the compounds

of the formula (I) have asymmetric centres, and thus are capable of existing in a number of stereoisomeric forms. The invention extends to each of these stereoisomeric forms, and to mixtures thereof. The different stereoisomeric forms may be separated one from the other by the usual methods.

The present invention further provides a process for the preparation of the compounds of the formula (I), which process comprises the cyclisation of a compound of formula (V):

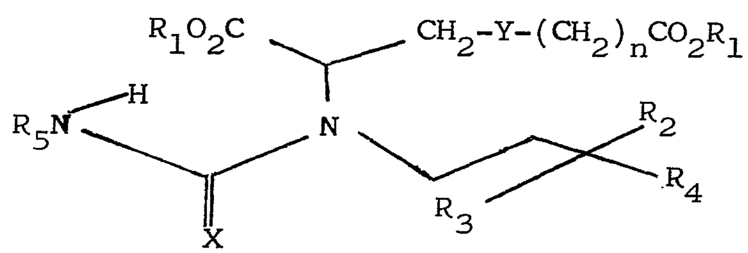

(V)

wherein the variable groups are as defined; and thereafter if desired or necessary converting Y, $R_1$ or $R_3$ in the thus formed compound into other variables Y, $R_1$ or $R_3$.

The compound of the formula (V) is conveniently prepared <u>in situ</u> during the reaction of a compound of the formula (VI):

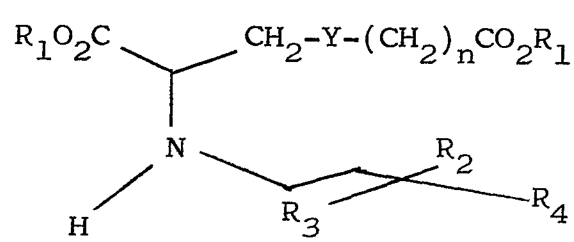

(VI)

wherein n, $R_1$, $R_2$, $R_3$ and $R_4$ are as defined, with $R_5NCX$, a preferred process of the invention.

The preferred process is suitably carried out under reflux in an inert solvent such as benzene or the like. It should be stated that when in this reaction $R_5$ is a sterically hindered group then this reaction may proceed only as far as the uncyclised compound of formula (V) in which case the necessary cyclisation of the compound (V) can be achieved with a strong base, such as sodium hydride or sodium ethoxide, in a dry organic solvent. Sodium ethoxide in benzene, or potassium t-butoxide in toluene, benzene, or hexamethylphosphoramide are suitable reagents.

The present invention also provides a further process for the preparation of compounds of the formula (I), which process comprises the reaction of a compound of the formula (VII):

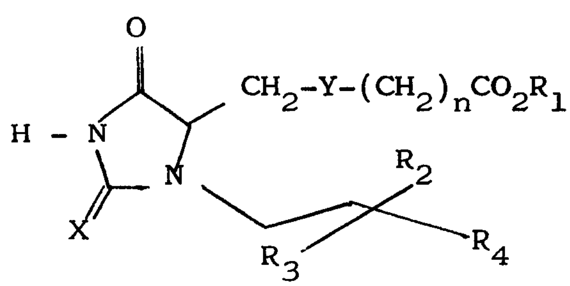

(VII)

wherein the variables are as defined in formula (I), with $R_5L$ wherein $R_5$ is as defined in formula (I) and L is a good leaving group. In such reactions it may be necessary to convert the compound of the formula (VII) first to an alkali metal salt at the 10-position (prostaglandin numbering).

This reaction is carried out under conventional conditions for substitution reactions. L may suitably be a halide.

Compounds of the formula (VII) may be prepared by the reaction of a compound of formula(VI) as defined with a salt $M^{+}\bar{C}NX$, wherein $M^{+}$ is a metal ion, in the presence of acid.

In this reaction the necessary acid may conveniently be provided by using an acid addition salt of the compound of formula (VI), such as the hydrochloride, or by carrying out the reaction in aqueous acid.

The subsequent conversion of a compound of the formula (I) to another compound of the formula (I) wherein Y, $R_1$ or $R_3$ are altered, when desired or necessary, may be achieved in conventional manner.

For example, if desired, compounds wherein Y is -C≡C- may be reduced to compounds wherein Y is -CH=CHin known manner. Suitably this reaction is carried out using catalytic hydrogenation, such as Lindlar catalysis.

Also if desired the group $R_1$ in the compound may be varied by conventional esterification and/or de-esterification reactions. Similarly, protected $R_3$ hydroxy moieties may be deprotected in conventional manner. For example when $R_3$ is a benxyloxy group, the benzyl group may readily be removed by acidic hydrolysis. Thus it may be seen that 'protected hydroxy' compounds of the formula (I) are useful intermediates in the preparation of the corresponding 'free hydroxy' compounds of the formula (I).

Also when a compound of the formula (I) contains an acidic hydrogen atom, a salt thereof may be prepared in conventional manner for example by reacting the compound of the formula (I) with the required base.

The compounds of formula (VI) are known compounds, or may be prepared in analogous manner to known compounds.

One suitable process for the preparation of a compound of formula (VI) comprises reacting a compound of formula (VIII):

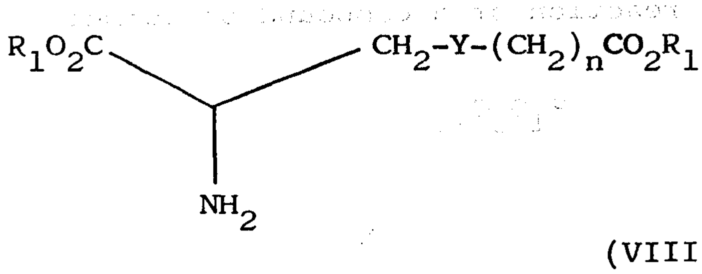

(VIII)

with a compound of formula (IX):

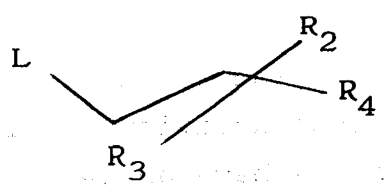

(IX)

This reaction is suitably carried out in an inert organic solvent, such as hexamethylphosphoramide or N,N-dimethylformamide, at room temperature, in the presence of a base, such as sodium carbonate or sodium hydride, and a source of alkali metal ions, such as an alkali metal halide. Suitable alkali halides include sodium iodide and lithium iodide.

The compounds of formula (VIII) may suitably be prepared by cleavage of a compound of formula (X):

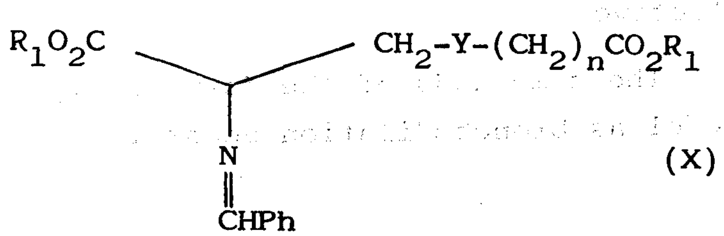

(X)

Suitably this cleavage is carried out with mild acid catalysis, for example by chromatography on acid washed silica gel.

These compounds of formula (X)can be prepared by reaction of a compound of formula (XI):

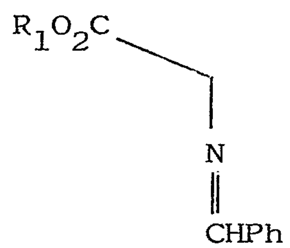

(XI)

with a compound of formula (XII) $L-CH_2-Y-(CH_2)_nCO_2R_1$ in normal manner for such alkylation reactions.

Compounds within the formula (I) have particularly useful pharmacological activity. For example compounds within the formula (I) have anti-gastric secretion activity, anti-ulcer activity, cardiovascular activity e.g. anti-hypertensive activity, platelet aggregation inhibition activity, affect the respiratory tract, e.g. bronchodilator activity, and have anti-fertility, smooth muscle and anti-arrhythmic activity.

Compounds of the formula (I) may accordingly be used in the treatment and prophylaxis of corresponding disorders in humans and animals.

In general it may be said that compounds within the formula (I) have a range of pharmacological activities similar to those shown by the natural prostaglandins, but that these activities tend to be rather more selective.

The compounds of the formula (I) are especially useful as bronchodilation agents.

The invention therefore also provides a pharmaceutical composition comprising a compound of the formula (I) and a pharmaceutically acceptable carrier.

The compounds of the formula (I) also have good stability.

Clearly the formulation of the said pharmaceutical composition will depend on the nature of the activity shown by the chosen compound of the formula (I), and on other factors such as a preference in a particular area of therapy for a particular mode of adminstration.

The composition may be in the form of tablets, capsules, powders, granules, lozenges or liquid preparations, such as oral or sterile parenteral solutions or suspensions.

Tablets and capsules for oral administration may be in unit dose presentation form, and may contain conventional excipients such as binding agents, fillers, tabletting lubricants, disintegrants, and acceptable wetting agents and the like. The tablets may be coated according to methods well known in normal pharmaceutical practice. Oral liquid preparations may be in the form of, for example, aqueous or oily suspensions, emulsions, syrups, or elixirs, or may be presented as a dry product for reconstiturion with water or other suitable vehicle before use. Such liquid preparations may contain conventional additives such as suspending agents, emulsifying agents, non-aqueous vehicles (which may include edible oils), preservatives, and if desired conventional flavouring or colouring agents, and the like.

For parenteral administration, fluid unit dosage forms are prepared utilising the compound of the formula (I) and a sterile vehicle. The compound, depending on the vehicle and concentration used, can be either

suspended or dissolved in the vehicle. In preparing solutions the compound can be dissolved for injection and filter sterilised before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

When appropriate, the compositions of this invention may be presented as aerosol for oral administration, or as a microfine powder for insufflation.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The invention also provides a method of treatment and/or prophylaxis of disorders in human being or animals which comprises the administration to the sufferer of an effective amount of a compound of the formula (I). Normally however the compounds will be used in the therapy of human disorders.

The following Examples illustrate the preparation of compounds of the formula (I) and their pharmacological properties.

EXAMPLE 1 (a)

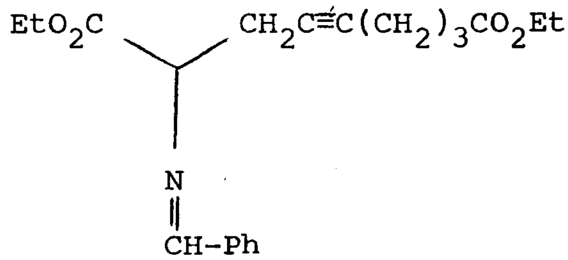

The N-benzylidene derivative of glycine ethyl ester prepared as described by G.Stork, et al, J. Org. Chem., 41, 3491 (1976) (5.70 g 0.03 mole) in dry tetrahydrofuran (25 ml) was added dropwise to a stirred suspension of potassium t-butoxide (3.60 g, 0.033 mole) in dry tetrahydrofuran (50 ml) under nitrogen at -78°. The solution immediately became bright red in colour.

After 30 minutes, sodium iodide (0.5 g) was added to the solution, followed by the dropwise addition of ethyl 7-bromo-hept-5-ynoate (6.95 g, 0.03 mole). The solution became pale yellow in colour as it was allowed to warm up to room temperature over 2 hours. The solution was stirred for a further 5 hours at room temperature. Saturated ammonium chloride solution (75 ml) was added, and the organic layer was extracted with ether, washed with brine (2 x 100 ml), dried ($Na_2SO_4$) and evaporated in vacuo below 30° to give ethyl 2-(benzyl ideneamino)-8-ethoxycarbonyl-oct-4-ynoate (7.5 g) as an orange oil.

EXAMPLE 1 (b)

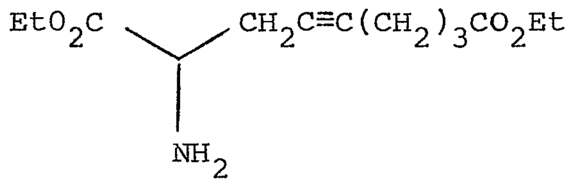

Ethyl 2-(benzylideneamino)-8-ethoxycarbonyl-oct-4-ynoate (7.5 g) was passed through acid-washed silica gel (Merck: Kieselgel 60) (75 g). After elution of benzaldehyde with pentane, elution with 95% ether/5% methanol gave ethyl 2-amino-8-ethoxycarbonyloct-4-ynoate (3 g) as a colourless oil.

EXAMPLE 1 (c)

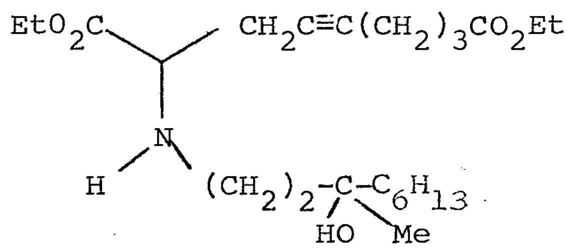

To a solution of ethyl 2-amino-8-ethoxycarbonyl-oct-4-ynoate (2.7 g, 0.01 mole) in hexamethylphosphoramide (10 ml) was added sodium carbonate (1.6 g, 0.015 mole), sodium iodide (1g, 0.007 mole) and 1-(p-toluene-sulphonyl)-3-methyl-nonan-3-ol (4.0 g, 0.011 mole) in hexamethylphosphoramide ( 10 ml), and the resultant mixture was stirred at room temperature for 70 hours.

The reaction mixture was then poured into water (200 ml) and extracted with ether (3x 200 ml). The combined organic extracts were washed with water (3 x 200 ml) and saturated sodium chloride solution (3 x 200 ml), dried ($Na_2SO_4$) and evaporated *in vacuo* to give an orange oil ( 6 g). This was chromatographed on silica gel (200 g) using chloroform as eluant to give ethyl 8-ethoxycarbonyl-2-[N-(3'-hydroxy-3'-methyl-*n*-nonyl)-amino]-oct-4-ynoate (2.7 g) as a yellow oil.

0006352

EXAMPLE 1 (d)

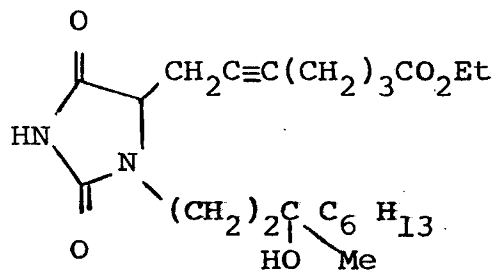

A solution of ethyl 8-ethoxycarbonyl-2-[N-(3'-hydroxy-3'-methyl-n-nonyl)-amino] oct-4-ynoate (4.11 g, 0.01 mole) in ethanol (20 ml) and 2N-hydrochloric acid (10 ml) was stirred and cooled in ice during the dropwise addition of potassium cyanate (1.62 g, 0.02 mole). The mixture was stirred at room temperature for 4 hours. The ethanol was evaporated in vacuo, water (100 ml) was added and the insoluble oil was extracted with ether (200 ml). The ethereal extract was washed with brine (100 ml), dried ($Na_2SO_4$) and evaporated in vacuo to give an orange oil (4 g).

The oil was heated at 120° for 4 hours to yield a dark orange gum (4 g), which was chromatographed on silica gel (Merck: Kieselgel 60) (150 gm) using chloroform and 1% methanol/chloroform as successive eluants to give 1-(3'-hydroxy-3'-methyl-n-nonyl)-5-(6"-ethoxycarbonyl-n-hex-2"-ynyl) hydantoin (2.5 g) as a yellow oil.

EXAMPLE 1 (e)

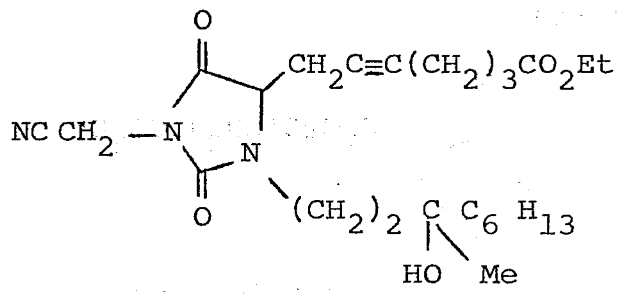

Compound 1

1-(3'-hydroxy-3'-methyl-n-nonyl)-5-(6"-ethoxycarbonyl-n-hex-2"-ynyl) hydantoin (2 g, 0.005 mole) in dry dimethylformamide (10 ml) was added to a stirred suspension of sodium hydride (0.165 g, 0.0055 mole; 80% dispersion in oil) under nitrogen at room temperature. The mixture was stirred for 3 hours.

Chloroacetonitrile (0.42 g, 0.0055 mole) in dry dimethylformamide (10 ml) was added dropwise to the stirred solution and the mixture was stirred for 6 hours at room temperature. The reaction mixture was poured into water (100 ml) and extracted with ether (2 x 100 ml). The combined organic solutions were washed with brine (2 x 100 ml), dried ($Na_2SO_4$) and evaporated in vacuo to give a yellow oil (2 g). The oil was chromatographed on silica gel (Merck: Kieselgel 60: 100 g) using chloroform, 1% methanol/ Chloroform, 2% methanol/chloroform as successive eluants to give 1-(3'-hydroxy -3'- methyl-n-nonyl)-3-cyanomethyl-5-(6" ethoxycarbonyl-n-hex-2"-ynyl) hydantoin (1 g) as a pale yellow gum.

| | | |
|---|---|---|
| IR ($cm^{-1}$) | 3450 | [OH] |
| | 1760, 1730 (broad) | $[-N-\overset{O}{\overset{\|}{C}}-N-\overset{O}{\overset{\|}{C}}-; CO_2Et]$ |
| NMR ($\tau$) | 8.1 to 7.2 | (brm, 6H, $C\underline{H}_2$, $C{\equiv}CC\underline{H}_2$, $C\underline{H}_2CO_2Et$) |
| | 7.3 | (bs, 1H, OH) |
| | 5.95 | (q, 2H, $CO_2CH_2$) |
| | 5.9 | (t, 1 H, N-CH) |
| | 5.65 | (S, 2H, $CH_2CN$) |

Example 2

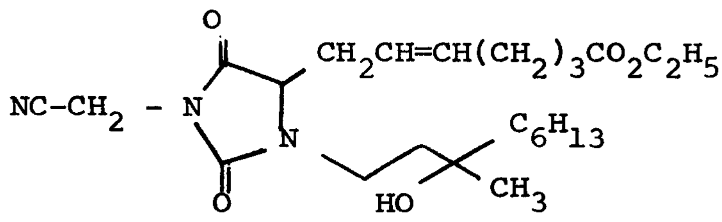

Compound 2

1-(3'-Hydroxy-3'-methyl-n-nonyl)-3-cyanomethyl-5-(6"-ethoxycarbonyl-n-hex-2"-ynyl) hydantoin (1 g) in methanol (30 ml) containing Pd/$CaCO_3$ (0.1 g) and quinoline (0.1 ml) was shaken in a hydrogen atmosphere at room temperature until uptake of hydrogen ceased. The mixture was filtered through Kieselguhr and the residue was washed with ether. The combined organic solutions were washed with dilute hydrochloric acid and with brine, then were dried and evaporated in vacuo to give 1-(3'-hydroxy-3'-methyl-n-nonyl)-3-cyanomethyl-5-(6"-ethoxycarbonyl-n-hex-2"-enyl) hydantoin (400 mg as a colourless gum).

Example 3

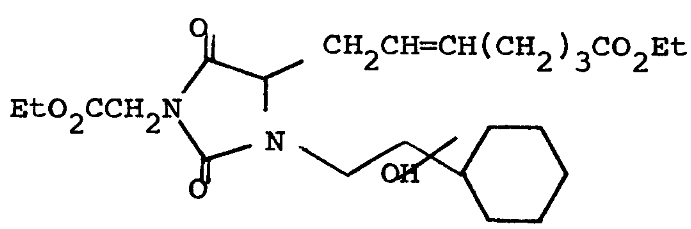

Compound 3

1-(3'-hydroxy-3'-cyclohexylbutyl)-5-(6"-ethoxycarbonyl-n-hex-2"-enyl) hydantoin (1.34 g, 3.3 m mol) in dry dimethylformamide (50 ml) was added in one portion to sodium hydride (80% dispersion in oil) (0.10 g, 3.3 m mol) in dry dimethylformamide (10 ml) at room temperature,

under nitrogen. After two hours at room temperature, a clear solution was formed. Ethyl bromoacetate (0.55 g, 3.3 m mol) was added in one portion. The mixture was stirred overnight and poured into brine (200 ml). The product was extracted with ether (3 x 200 ml) and the combined extracts washed with brine and dried over sodium sulphate. The product was chromatographed on silica gel (40 g) using ethyl acetate, hexane (50:50) as eluent. This gave 1-(3'-hydroxy-3'-cyclohexylbutyl)-3-(ethoxycarbonylmethyl)-5-(6"-ethoxycarbonyl-n-hex-2"-enyl) hydantoin (1.35 g) as an orange oil.

I.R. ($\nu_{max}$) $cm^{-1}$ 3400 (OH), 1710 and 1770 (N-C(=O)-N-C(=O)), 1735 (terminal $CO_2Et$), 1745 (C=O in $EtO_2CCH_2N<$ ).

N.M.R. ($\tau$) 4.55 (2H, m, olefinic protons), 5.78 (s, C(=O)-$CH_2$-N)/ superimposed on, 5.5-6.7 (7H, m, 2 x $CO_2$-$CH_2$-Me, C(=O)-$CH_2$-N, C(=O)-CH-N), 8.74 (6H, t, $OCH_2$-$CH_3$), 8.84 (3H, s, $CH_3$-C-OH).

H.R.M.S. m/e ($M^*-H_2O$), $C_{26}H_{40}N_2O_6$ requires 476.2884
found 476.2884
($M^*$ at 494)

Example 4

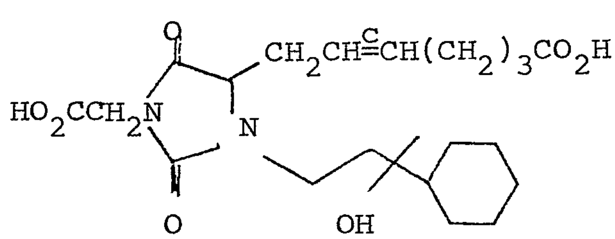

Compound 4

1-(3'-hydroxy-3'-cyclohexylbutyl)-3-(ethoxycarbonylmethyl)-5-(6"-ethoxycarbonyl-n-hex-2"-enyl) hydantoin (1.26 g, 2.6 m mol) in methanol (24 ml) was refluxed overnight with 10% aqueous potassium carbonate (24 ml). The mixture was cooled, and ice (15 g) added. The solution was acidified to pH 1 with concentrated hydrochloric acid and extracted with ether (3 x 100 ml). The combined ether extracts were extracted with 5% sodium bicarbonate solution (3 x 100 ml). The combined bicarbonate layers were washed with ether (50 ml), acidified to pH 1.5 with concentrated hydrochloric acid and extracted with ether (3 x 100 ml). The combined ether layers were washed with saturated brine (3 x 50 ml) and dried over sodium sulphate. Removal of the solvent under reduced pressure gave 1-(3'-hydroxy-3'-methyl-cyclohexylbutyl)-3-(carboxymethyl)-5-(6"-carboxy-n-hex-2"-enyl) hydantoin(0.72 g) as a pale reddish brown gum.

I.R. ($\nu_{max}$) $cm^{-1}$ 3400-2500 (OH, $CO_2H$), 1765, 1700 (N-C(=O)-N-C(=O)), 1710 ($CO_2H$).

N.M.R.($\tau$) 2.10 (3H, bs, OH, 2 x $CO_2H$), 4.6 (2H, m, vinyl protons), 5.6-6.1 (3H, $HO_2C-CH_2$-N, O=C–N–H ).

Characterising Data for Compound 2

N.M.R. ($\tau$ $CDCl_3$): 8 to 7.2, m, 7H, ($CH_2$-CH=CH-$CH_2$; $CH_2CO_2C_2H_5$; OH);
6.5, brm, 2H, ($NCH_2$);
5.8, q, 2H, superimposed on t, 1H, ($CO_2CH_2CH_3$; NCH);
4.6, m, 2H, (CH=CH).

Mass spec: $C_{24}H_{39}N_3O_5$ ($M^*$)
required: 449.2887
found: 449.2912.

Pharmacological Data Section

Bronchodilator Activity

(a) The Compounds were examined for their ability to inhibit 5-hydroxytryptamine induce bronchoconstriction in the anaesthetised, artificially respired guinea-pig (Konzett-Rossler preparation).

Compound 2 had an $ED_{50}$ of 1.1 μg/kg i.v.

(b) The Compounds were also examined for their ability to protect conscious guinea-pigs against bronchoconstriction induced by an histamine aerosol (Herxheimer test). The Compounds were administered by aerosol.

The aerosol was produced in the following manner.

Water soluble compounds were dissolved in normal saline at a concentration of 1 mg/ml. Water insoluble compounds were dissolved in ethanol at a concentration of 10 mg/ml. These solutions were then diluted to the test concentrations with normal saline.

Compound 2 at 10 μg/ml gave a 178% increase in preconvulsive coughing time 2 minutes after dosing, and a 39% increase after 10 minutes.

What we claim is:

1. A compound of the formula (I):

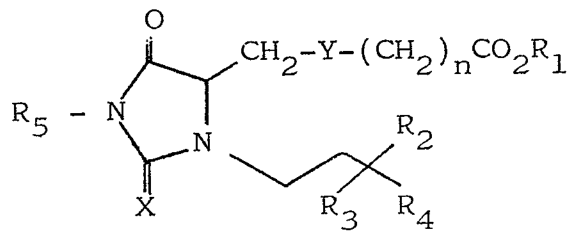

(I)

wherein:

X is O or S;

Y is -CH=CH- or -C≡C-;

n is 1 to 5;

$R_1$ is hydrogen or $CO_2R_1$ represents an ester group in which the $R_1$ moiety contains from 1 to 12 carbon atoms;

$R_2$ is hydrogen or $C_{1-4}$ alkyl;

$R_3$ is hydroxy or protected hydroxy;

$R_4$ is $C_{1-9}$ alkyl, $C_{3-8}$ cycloalkyl or $C_{3-8}$ cycloalky-$C_{1-6}$ alkyl; or

$R_2$ and $R_4$ taken with the carbon atom to which they are joined represent a $C_{5-8}$ cycloalkyl group; and

$R_5$ is $C_{1-6}$ alkyl substituted by one nitro, hydroxy, $C_{1-6}$ alkoxy, $CO_2A$, $(CO_2A)_2$, CN or halogen group; $C_{5-8}$ cycloalkyl; or $CO_2A$; in $R_5$ when present A is hydrogen or $CO_2A$ represents an ester group in which the A moiety contains from 1 to 12 carbon atoms; and salts thereof.

2. A compound according to claim 1, wherein X is O.

3. A compound according to claim 1 or claim 2, wherein n is 3.

4. A compound according to claim 1, 2 or 3, wherein $R_1$ is hydrogen or $C_{1-6}$ alkyl.

5. A compound according to claim 1, 2, 3 or 4, wherein $R_2$ is methyl.

6. A compound according to any one of the previous claims wherein $R_3$ is hydroxy.

7. A compound according to any one of the previous claims, wherein $R_4$ is a $C_{4-9}$ alkyl group.

8. A compound according to claim 7, wherein $R_4$ is n-hexyl.

9. A compound according to claim 7, wherein $R_4$ is $-C(CH_3)C_4H_9$.

10. A compound according to any one of the claims 1 to 6, wherein $R_4$ is or contains a cyclohexyl moiety.

11. A compound according to any one of the preceding claims, wherein $R_5$ is methylene substituted by one $C_{1-6}$ alkoxy, $CO_2A$, $(CO_2A)_2$ or CN.

12. A compound according to claim 11, wherein $R_5$ is $-CH_2CN$.

13. A pharmaceutical composition comprising a compound according to any one of the preceding claims and a pharmaceutically acceptable carrier.

14. A process for the preparation of a compound according to claim 1, which process comprises the cyclisation of a compound of formula (V):

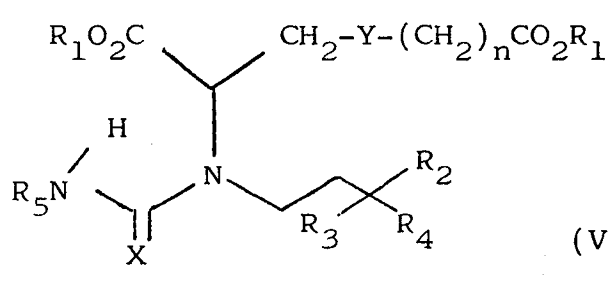

(V)

wherein the variable groups are as defined; and thereafter if desired or necessary converting Y, $R_1$ or $R_3$ in the thus formed compound into other variables Y, $R_1$ or $R_3$.

15. A process according to claim 14, wherein the compound of formula (V) is prepared <u>in situ</u> during the reaction of a compound of formula (VI):

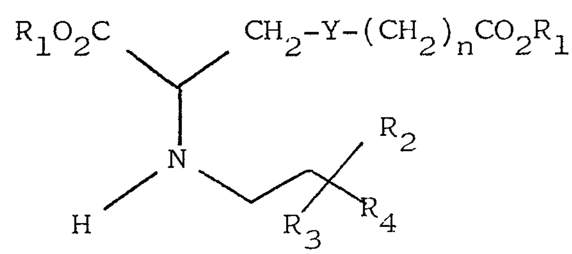

(VI)

with $R_5NCX$.

16. A process for the preparation of a compound according to claim 1, which process comprises reacting a compound of formula (VII):

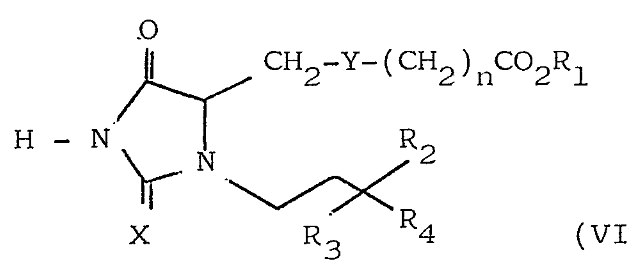

(VII)

with $R_5L$, wherein L is a good leaving group.

0006352

European Patent Office

# EUROPEAN SEARCH REPORT

Application number

EP 79 301 143.8

| Category | Citation of document with indication, where appropriate, of relevant passages | Relevant to claim |
|---|---|---|
| D | DE - A - 2 724 948 (THE WELLCOME FOUNDATION)<br>* claims 1, 13, 22, 25 * | 1, 13-16 |
| D,P | DE - A - 2 755 771 (BEECHAM GROUP)<br>* claims 1, 30 to 33 * | 1, 13-15 |
| P | EP - A - 0 001 238 (THE WELLCOME FOUNDATION)<br>* claims 1, 8, 11 * | 1,13, 14 |

**CLASSIFICATION OF THE APPLICATION (Int. Cl.3)**

C 07 D 233/76
A 61 K 31/415

**TECHNICAL FIELDS SEARCHED (Int.Cl.3)**

A 61 K 31/415
C 07 D 233/76
C 07 D 233/86

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 12-09-1979 | FROELICH |